# EUROPÄISCHE PATENTANMELDUNG

(11) **EP 2 005 845 A1**
(43) Veröffentlichungstag der Anmeldung: **24.12.2008**
(21) Anmeldenummer: 07110900.3
(22) Anmeldetag: 22.06.2007
(51) Int. Cl.: A23L 2/385, A23L 1/05, A23L 1/0522, A23L 1/054, A23L 1/0526, A61K 9/00

(54) **Disperser Grundstoff**

(71) Anmelder: Döhler GmbH, 64295 Darmstadt (DE)
(72) Erfinder: Bonerz, Daniel, 64625 Bensheim (DE); Füsser, Hans, 64389 Münster (DE); Wydra, Markus, 64673 Zwingenberg (DE); Krug, Harald, 69231 Rauenberg (DE); Euler-Horn, Elke, 64367 Mühltal (DE)
(74) Vertreter: Schreiber, Christoph

(57) **Zusammenfassung**

Verfahren zur Herstellung einer Grundstoffdispersion umfassend den Schritt
- Vermischen von
- 0,1 bis 15 Gew.-% eines oder mehrerer Verdickungsmittel mit
- 0,1 bis 70 Gew.-% von mindestens zwei zu dispergierenden Grundstoffinhaltsstoffen
- gegebenenfalls Hilfsstoffen und

- Wasser.

## Beschreibung

Die vorliegende Erfindung betrifft ein Verfahren zur Herstellung von Grundstoffdispersionen sowie dadurch erhältliche Grundstoffdispersionen und ihre Verwendung.

Viele industriell hergestellte Lebensmittel, beispielsweise Getränke, werden hergestellt durch das Vermischen verschiedener industriell vorgefertigter Substanzen, beispielsweise durch das Vermischen von Zucker, Zitronensäure, Saft, Farbstoff, Aroma und Wasser, um eine Limonade zu erhalten. Industriell wird ein Großteil der marktbekannten Getränkeprodukte in einem ersten Schritt in Form eines konzentrierten Getränkegrundstoffs hergestellt. Diese Grundstoffe werden vor Füllung in das Verkaufsgebinde auf Trinkstärke verdünnt und optional auch mit weiteren Getränkeinhaltsstoffen versetzt. Soweit hierzu trockene, pulverförmige Substanzen eingesetzt werden, werden diese im Regelfall abgewogen und dann in einen Mischer gegeben bzw. gelöst oder in etwas Wasser vorgelöst zugegeben.

Die Handhabung trockener Produkte ist technisch schwierig. Einige dieser Substanzen weisen darüber hinaus eine limitierte Löslichkeit auf, was ihre Handhabung in Herstellprozessen schwierig machen kann.

Die WO 95/15697 beschreibt eine wässrige Suspension eines Dipeptidsüßstoffes (Aspartam) mit einem "stabilizing flow agent". Da in der Lebensmittelindustrie fast ausschließlich Kombinationen der bekannten Süßstoffe zum Einsatz kommen, ist der Einsatz der Aspartamsuspension der WO 95/15697 nur in Ausnahmefällen wirtschaftlich und technologisch sinnvoll. Es besteht daher weiterhin ein Bedarf nach einfachen Prozessen zur Herstellung von Getränken und Lebensmitteln, die die genannten Nachteile des Standes der Technik überwinden.

Gelöst wird die Aufgabe durch ein Verfahren zur Herstellung einer Grundstoffdispersion umfassend den Schritt
- Vermischen von
   - 0,1 bis 15 Gew.-% eines oder mehrerer Verdickungsmittel mit
   - 0,1 bis 70 Gew.-% von mindestens zwei zu dispergierenden Grundstoffinhaltsstoffen
   - gegebenenfalls Hilfsstoffen und
   - Wasser.

Erfindungsgemäß werden also Mischungen von mindestens zwei Grundstoffinhaltsstoffen hergestellt, die durch Verdickungsmittel in einer Dispersion stabilisiert werden. Solche erfindungsgemäßen Dispersionen sind - obwohl sie hohe Gehalte an Inhaltsstoffen aufweisen - im Regelfall fließ- und pumpfähig und daher sehr einfach in Getränke bzw. Nahrungsmittel einzubringen.

In einer besonderen Ausführungsform wird
a) zunächst eine Gelphase, enthaltend das oder die Verdickungsmittel, Wasser sowie gegebenenfalls Hilfsstoffe, hergestellt und,
b) anschließend eine Dispersion durch Vermischen der Gelphase aus Schritt a) mit mindestens zwei zu dispergierenden Grundstoffinhaltsstoffen hergestellt.

Die erfindungsgemäß eingesetzten Verdickungsmittel werden insbesondere ausgewählt aus Carboxymethylcellulose, Xanthan, Johannisbrotkernmehl, Guarkernmehl, Tarakernmehl, Konjakmehl, Pektin (hochverestert, niedrigverestert, amidiert), Stärke, Gellan und Mischungen davon.

In einer bevorzugten Ausführungsform beträgt die Menge an Verdickungsmitteln 0,5 bis 5 Gew.-%.

Der bevorzugte Gehalt an zudispergierenden Grundinhaltsstoffen beträgt 0,1 bis 40%, mehr bevorzugt 1 bis 30%.

Weiterhin wird bevorzugt, dass von den mindestens zwei zu dispergierenden Grundstoffinhaltsstoffen jeder mehr als mindestens 0,5 Gew.-% in der Grundstoffdispersion aufweist.

Die zwei oder mehr zu dispergierenden Grundstoffinhaltsstoffe können einzeln oder vorgemischt in die Gelphase eingebracht werden.

Als zu dispergierende Grundstoffinhaltsstoffe eignen sich insbesondere Süßstoffe, Konservierungsstoffe, Mineralien, Geschmacksverstärker, Farbstoffe, Pigmente, funktionelle Inhaltsstoffe, Ballaststoffe und/oder pflanzliche Extrakte. Als zu dispergierende Grundinhaltsstoffe sind alle Inhaltsstoffe geeignet, die in einer solchen Konzentration in die Dispersion eingebracht werden sollen, dass sie nicht mehr komplett in Lösung gehen können. Über die Kombination aus mindestens zwei nicht (mehr) vollständig im dispersen Grundstoff löslichen Inhaltsstoffen kann dieser zusätzlich eine Vielzahl weiterer durchaus gänzlich löslicher Grundstoffinhaltsstoffe enthalten.

Typische zu dispergierende Grundstoffinhaltsstoffe zeigen eine schlechte Löslichkeit, dass heißt eine nicht vollständige Löslichkeit bei einer vorgegebenen Ausgiebigkeit (Verhältnis der verwendeten Grundstoffmenge zur Menge an erhaltenen zugehörigen Fertiggetränken) . Sollen solche Substanzen in Mengen in Grundstoffdispersionen eingebracht werden, in denen sie nicht mehr löslich sind, werden sie die erfindungsgemäß eingesetzten "zu dispergierenden Grundstoffinhaltsstoffe". Es gibt jedoch auch Substanzen, die eine höhere Löslichkeit aufweisen, jedoch in Konzentrationen, die über ihrer Löslichkeit liegen, in die Grundstoffdispersion eingebracht werden sollen. Auch solche sind von den erfindungsgemäßen "zu dispergierenden Grundstoffinhaltsstoffen" umfasst.

Erfindungsgemäß werden zwei oder mehr zu dispergierende Grundstoffinhaltsstoffe eingesetzt. Es können auch drei, vier, fünf oder mehr Stoffe kombiniert werden. In bevorzugter Weise enthält die Grundstoffdispersion bereits alle wesentlichen Inhaltsstoffe, um durch Zugabe einer wässrigen Komponente (beispielsweise Wasser, Milch, Tee, Joghurt etc.) zu einem verzehrbaren Produkt zu gelangen.

Ein Einsatzbereich des erfindungsgemäßen Verfahrens ist die Herstellung von Grundstoffdispersionen, die Süßstoffe, auch als "high-intensity-sweeteners" bezeichnet, enthalten. Viele dieser Substanzen weisen mäßige Löslichkeiten auf.

Geeignete Süßungsmittel für das erfindungsgemäße Verfahren sind insbesondere Süßstoffe, wie Na-Cyclamat, Na-Saccharin, Aspartam, Acesulfam K, Aspartam-Acesulfam-Salz, Sucralose, Neotame, Thaumatin, Neohesperidin DC, Mogroside (Luo han Guo), des weiteren Zuckeralkohole (wie Sorbit, Xylit, Mannit), Fructose, Isomaltulose, Trehalose, Tagatose, High Fructose Corn Syrup, Glucose, Invertzuckersirup, Zucker, Zuckersirup. Ein bevorzugter Bereich für den Gehalt an der Mischung aus mindestens zwei Süßungsmitteln beträgt 5 bis 35 Gew.-%.

Die Verwendung einer Süßungsmittelmischung, die kein Aspartam und insbesondere keine Dipeptidsüßungsmittel enthält, ist ebenfalls eine Ausführungsform der Erfindung.

Üblicherweise schließt sich an den Schritt a) des erfindungsgemäßen Verfahrens mindestens einer der Schritte Turraxen, Homogenisieren oder Vermahlen an. Auch andere dem Fachmann bekannte Dispergierverfahren können eingesetzt werden.

Je nach Anwendungszweck kann es auch sinnvoll sein, dass sich an den Schritt a) oder an den eben beschriebenen Schritt des Dispergierens noch ein Pasteurisationsschritt oder eine andere thermische Behandlung anschließt.

Auch oder ausschließlich an den Schritt b) des erfindungsgemäßen Verfahrens kann sich ein Schritt wie Rühren, Homogenisieren, Vermahlen, Turraxen oder der Einsatz anderer, dem Fachmann bekannter Dispergierverfahren anschließen.

Als Hilfsstoffe können beispielsweise Säuerungsmittel, Konservierungsmittel, Farbstoffe und/oder Mischungen hiervon eingesetzt werden. Selbstverständlich ist auch die Kombination verschiedener Säuerungsmittel, etc. möglich.

Weiterhin ist es möglich zusätzliche Inhaltsstoffe vorzusehen, beispielsweise Ballaststoffe, Prebiotika, Probiotika, Aromen, Geschmacksverstärker, funktionelle Inhaltsstoffe wie Coffein, Taurin, D-Glucurono-γ-Lacton, Omega-3-Fettsäuren, pharmazeutisch wirksame Substanzen und/oder Mischungen der Vorgenannten.

Gegenstand der Erfindung ist auch eine Grundstoffdispersion, die durch das erfindungsgemäße Verfahren erhältlich ist.

Ein weiterer Gegenstand ist das Lebensmittel, erhältlich durch Vermischen der dispersen Grundstoffdispersion mit weiteren Inhaltsstoffen, insbesondere Wasser, sowie die Verwendung der erfindungsgemäßen Grundstoffdispersion zur Herstellung von Lebensmitteln, insbesondere von Getränken.

Gegenüber den üblichen Pulvern hat die erfindungsgemäße Grundstoffdispersion den Vorteil, dass sie ein staubfreies Einarbeiten bei der Anwendung ermöglicht. Darüber hinaus kann fallweise durch die bessere Löslichkeit (z. B. aufgrund der Vorbenetzung) die notwendige Zeit für das Einarbeiten schwerlöslicher Inhaltsstoffe im Endprodukt verkürzt werden.

Überraschenderweise zeigt sich, dass die erfindungsgemäßen, komplexer als der Stand der Technik aufgebauten Grundstoffdispersionen eine erhöhte Stabilität aufweisen. Bei einer Lagerung für 70 Tage bei Raumtemperatur und pH = 2,8 kommt es nur zu einem Abbau von etwa 5% der enthaltenen Süßstoffe wie Aspartam oder Neotam, während bei einer üblichen flüssigen Süßung ein Abbau von etwa 25 bis 30% erfolgt.

Aufgrund der hohen Konzentration der Inhaltsstoffe im dispersen Süßungsmittel ergeben sich optional Vorteile bei der Lagerung und dem Transport. Es zeigt sich, dass die erfindungsgemäße Grundstoffdispersion in einem Großteil der Fälle nicht gekühlt gelagert werden muss und - zumindest über einen Zeitraum von 3 Monaten - mikrobiologisch stabil ist.

Aufgrund der sehr hohen Einträge, insbesondere an schwerlöslichen Inhaltsstoffen ergibt sich eine hohe Ausgiebigkeit. Statt der üblichen Dosierung von zum Beispiel Flüssigsüßungen von 20 : 1000 und größer im Endgetränk genügt eine Dosierung von etwa 1,5 - 5 : 1000.

Grundsätzlich ist auch die Formulierung (schwerlöslicher) pharmakologisch aktiver Substanzen durch das erfindungsgemäße Verfahren denkbar. Die erfindungsgemäße Grundstoffdispersion ist sowohl geeignet zur Herstellung von Arzneimitteln, von denen beispielsweise noch weitere Inhaltsstoffe, wie Süßungsmittel etc. eingebracht werden müssen, als auch für Arzneimittel, bei denen der Wirkstoff selbst schwer löslich ist und durch das erfindungsgemäße Verfahren in eine geeignete Dispersionsform gebracht werden kann, die entweder beim pharmazeutischen Hersteller oder beim Patienten nur noch entsprechend ausgemischt beziehungsweise verdünnt bzw. in der dispersen Form konsumiert werden muss.

### Beispiele:

### a) Disperse 4er-Süßung, Dosage von 1,5 g/L (9 % Zuckeräquivalente) (entspricht 8,5 g/L der analogen Flüssigsüßung)

### Durchführung:

**Produktion des Gels für disperse Süßungen (10 kg):**

| | |
|---|---|
| Stadtwasser (50 - 60 °C) | 8,027 kg |
| Zitronensäure (1 : 1) | 0,800 kg |
| K-Sorbat (10 %) | 0,067 kg |
| Xanthan | 0,020 kg |
| Johannisbrotkernmehl | 0,085 kg |
| Fahrwasser | 1,000 kg |

### Arbeitsanweisung:

- Wasser (50 - 60 °C), und K-Sorbat vorlegen, Zitronensäure einrühren
- Xanthan und Johannisbrotkernmehl mischen und in Lösung einrühren bis vollständig gelöst
- Homogenisieren
- Anschließend Pasteurisation
- Abkühlen auf ca. 10°C
   ⇒ Gelkomponente für disperse Süßung

### Einbringen der Süßstoffe:

- 10 kg Gel
- 2,270 kg Na-Cyclamat
- 0,406 kg Acesulfam K
- 0,507 kg Na-Saccharin
- 0,359 kg Aspartam

Die Süßstoffe werden in das kalte Gel eingerührt und optional kurz geturraxt.

Anschließend wird die Süßstoff-Gellösung 1x homogenisiert (200 / 40). Ohne weitere Pasteurisation war das Produkt stabil.

### b) Disperse 3er-Süßung ohne Cyclamat, Dosage von 2,5 g/L (entspricht etwa 10 g/L der analogen Flüssigsüßung)

### Durchführung:

**Produktion des Gels für disperse Süßungen (10 kg):**

| | |
|---|---|
| Stadtwasser (50 - 60 °C) | 8,027 kg |
| Zitronensäure (1 : 1) | 0,800 kg |
| K-Sorbat (10 %) | 0,067 kg |
| Xanthan | 0,020 kg |
| Johannisbrotkernmehl | 0,085 kg |
| Fahrwasser | 1,000 kg |

### Arbeitsanweisung:

- Wasser (50 - 60 °C), und K-Sorbat vorlegen, Zitronensäure einrühren
- Xanthan und Johannisbrotkernmehl mischen und in Lösung einrühren bis vollständig gelöst
- Homogenisieren
- Anschließend Pasteurisation
- Abkühlen auf ca. 10 °C
   ⇒ Gelkomponente für disperse Süßung

### Einbringen der Süßstoffe:

- 91,28 g Gel
- 3,60 g Acesulfam K
- 1,52 g Na-Saccharin
- 3,60 g Aspartam

Die Süßstoffe werden in das kalte Gel eingerührt und kurz geturraxt. Anschließend wird die Süßstoff-Gellösung 1x homogenisiert.

Es war keine Pasteurisation erforderlich.

### c)Einfluss von Aspartam in gelöster Form auf die Viskosität des Gels

Es wurde analog der oben genannten Beispiele ein Gel produziert und die Viskosität bei 20 °C bei Scherraten von 10 - 100 1/s gemessen. Zusätzlich wurde ein zweites Gel mit gleichen Mengen an Verdickungsmitteln produziert, nur darüber hinaus 1,5 % Aspartam hinzu gegeben und in der Hitze gelöst. Das Aspartam bleibt bei der Messtemperatur (20 °C) in der angegebenen Menge komplett gelöst. (Analog wurde der Versuch mit Acesulfam K durchgeführt).

**Ergebnis der Viskositätsmessungen:**

| | Viskosität [Pas] | Viskosität [Pas] |
|---|---|---|
| | (Scherrate 10 [1/s]) | (Scherrate 100 [1/s]) |
| Gel 1 | 0,46 | 0,20 |
| Gel 1 + 1,5 % Aspartam | 2,70 | 0,62 |
| Gel 2 | 0,81 | 0,31 |
| Gel 2 + 1,5 % Acesulfam K | 0,59 | 0,23 |

Es zeigt sich eine deutlich erhöhte Viskosität des gleichen Systems durch Aspartamzusatz, nicht durch Acesulfam K.

**Disperser Grundstoff Typ Cola**

| **"Cola Gel**" | |
|---|---|
| Wasser | 447,7 g |
| Karamel Kulör | 990,0 g |
| Natürliche Aromen Typ Cola | 10,9 g |
| Kaliumsorbat (10 %ig) | 30,0 g |
| Trinatriumcitrat | 106,0 g |
| o-Phosphorsäure 75 % | 63,0 g |
| Koffein wasserfrei | 64,0 g |
| Xanthan | 7,4 g |
| CMC | 11,0 g |

Nach dem Zusammenrühren der Substanzen ist das Koffein nicht komplett gelöst, sondern homogen in "Cola-Gel" verteilt.

Anschließend erfolgt das Einrühren von Süßstoffen nach folgender Rezeptur:

| | |
|---|---|
| Cola Gel | 1520 g |
| Aspartam | 90 g |
| Acesulfam K | 70 g |
| Na-Cyclamat | 155 g |
| Fahrwasser (kalt) | 165 g |

Nach dem Einrühren erfolgt ein Homogenisationsschritt und der disperse Cola-Grundstoff ist fertig.

### Dosage: 3 : 1000 + Säurekomponente 1,5 : 1000

Im Vergleich normale Rezeptur:
Farb-Aroma-Komponente (1,5 : 1000)
Säure-Koffein-Komponente (0,8 : 1000)
Süßungskomponente (8,5 : 1000)
Trinatriumcitrat (0,25 g/L, als Feststoff zugegeben).

## Patentansprüche

1. Verfahren zur Herstellung einer Grundstoffdispersion umfassend den Schritt
- Vermischen von
- 0,1 bis 15 Gew.-% eines oder mehrerer Verdickungsmittel mit
- 0,1 bis 70 Gew.-% von mindestens zwei zu dispergierenden Grundstoffinhaltsstoffen
- gegebenenfalls Hilfsstoffen und
- Wasser.

2. Verfahren nach Anspruch 1, **dadurch gekennzeichnet, dass**
a) zunächst eine Gelphase enthaltend das oder die Verdickungsmittel, Wasser sowie gegebenenfalls Hilfsstoffe hergestellt wird,
b) anschließend eine Dispersion durch Vermischen der Gelphase aus Schritt a) mit mindestens zwei zu dispergierenden Grundstoffinhaltsstoffen hergestellt wird.

3. Verfahren nach Anspruch 1 oder 2, **dadurch gekennzeichnet, dass** die Menge an Verdickungsmitteln 0,5 bis 5 Gew.-% beträgt.

4. Verfahren nach Anspruch 2 oder bis 3, **dadurch gekennzeichnet, dass** die zwei zu dispergierenden Grundstoffinhaltsstoffe vor dem Einbringen in die Gelphase miteinander gemischt werden.

5. Verfahren nach mindestens einem der Ansprüche 2 bis 4, **dadurch gekennzeichnet, dass** sich an den Schritt a) mindestens ein Durchmischungsschritt wie Turraxen, Homogenisieren, Vermahlen anschließt.

6. Verfahren nach mindestens einem der Ansprüche 2 bis 5, **dadurch gekennzeichnet, dass** sich an den Schritt a) oder den Schritt gemäß Anspruch 4 eine thermische Behandlung wie Pasteurisation anschließt.

7. Verfahren nach mindestens einem der Ansprüche 2 bis 6, **dadurch gekennzeichnet, dass** sich an den Schritt b) mindestens ein Durchmischungsschritt wie Rühren, Homogenisieren, Vermahlen, Turraxen anschließt.

8. Verfahren nach mindestens einem der Ansprüche 1 bis 7, **dadurch gekennzeichnet, dass** die Verdickungsmittel ausgewählt werden aus Carboxymethylcellulose, Xanthan, Johannisbrotkernmehl, Guarkernmehl, Tarakernmehl, Konjakmehl, Pektin (hochverestert, niedrigverestert, amidiert), Stärke, Gellan und Mischungen davon.

9. Verfahren nach einem der Ansprüche 1 bis 8, **dadurch gekennzeichnet, dass** die zu dispergierenden Grundstoffinhaltsstoffe ausgewählt werden aus Süßungsmitteln, Konservierungsstoffen, Mineralien, Geschmacksverstärkern, Farbstoffen, Pigmenten, funktionelle Inhaltsstoffe, Ballaststoffe und/oder pflanzlichen Extrakten.

10. Verfahren nach Anspruch 9, **dadurch gekennzeichnet, dass** die Süßungsmitteln ausgewählt werden aus Na-Cyclamat, Na-Saccharin, Aspartam, Acesulfam K, Aspartam-Acesulfam-Salz, Sucralose, Neotame, Thaumatin, Neohesperidin DC, Mogroside (Luo han Guo), Zuckeralkohole (wie Sorbit, Xylit, Mannit), Fructose, Isomaltulose, Trehalose, Tagatose, High Fructose Corn Syrup, Glucose, Invertzuckersirup, Zucker, Zuckersirup.

11. Verfahren nach mindestens einem der Ansprüche 1 bis 10, **dadurch gekennzeichnet, dass** als Hilfsstoffe Säuerungsmittel und Säureregulatoren, Konservierungsmittel, Farbstoffe und/oder Mischungen davon eingesetzt werden.

12. Verfahren nach mindestens einem der Ansprüche 1 bis 11, **dadurch gekennzeichnet, dass** als weitere Inhaltsstoffe Ballaststoffe, Prebiotika, Probiotika, Aromen, Geschmacksverstärker, funktionelle Inhaltsstoffe wie Omega-3-Fettsäuren, pharmazeutisch wirksame Substanzen und/oder Mischungen eingesetzt werden.

13. Grundstoffdispersion erhältlich durch das Verfahren nach einem der Ansprüche 1 bis 12.

14. Lebensmittel, insbesondere Getränk, erhältlich durch Vermischen der Grundstoffdispersion nach Anspruch 13 mit weiteren Inhaltsstoffen, insbesondere Wasser oder Milch.

15. Verwendung der Grundstoffdispersion nach Anspruch 13 zur Herstellung von Lebensmitteln, insbesondere von Getränken.

16. Arzneimittel, erhältlich durch Vermischen der Grundstoffdispersion nach Anspruch 13 mit weiteren Inhaltsstoffen.
